(19) Europäisches Patentamt
European Patent Office
Office européen des brevets

(11) Publication number: 0 102 194 B1

# (12) EUROPEAN PATENT SPECIFICATION

(45) Date of publication of patent specification: 22.10.86

(51) Int. Cl.[4]: C 07 D 205/04, A 61 K 31/395

(21) Application number: 83304348.2

(22) Date of filing: 27.07.83

## (54) 3-Phenoxy-1-azetidinecarboxamides and their use and preparation.

(30) Priority: 19.08.82 US 409476

(43) Date of publication of application: 07.03.84 Bulletin 84/10

(45) Publication of the grant of the patent: 22.10.86 Bulletin 86/43

(84) Designated Contracting States: AT BE CH DE FR GB IT LI LU NL SE

(56) References cited:
GB-A- 872 447
US-A-4 226 861

HOUBEN-WEYL, "Methoden der organischen Chemie, 4th edition, vol. VIII, 1952 GEORG THIEME, Verlag Stuttgart, page 153

**The file contains technical information submitted after the application was filed and not included in this specification**

(73) Proprietor: A.H. ROBINS COMPANY, INCORPORATED
1407 Cummings Drive P.O. Box 26609
Richmond Virginia 23261 (US)

(72) Inventor: Teng, Lina Chen
7638 Redbud Road
Richmond Virginia 23235 (US)

(74) Representative: Sheard, Andrew Gregory et al
Kilburn & Strode 30, John Street
London WC1N 2DD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 194 B1

**Description**

The present invention relates to novel 3-phenoxy-1-azetidinecarboxamides which exhibit anticonvulsant activity in animals and are effective in the treatment of epilepsy in humans.

N-loweralkyl-3-phenoxy-1-azetidinecarboxamides are disclosed in US—A—4226861 as having anti-convulsant activity and useful in the treatment of epilepsy.

The compounds of the present invention were discovered as metabolites in the bloodstream of animals treated with the foregoing N-loweralkyl compounds and have been found to have greater longevity in the bloodstream and greater persistence in their anti-convulsant effect than the corresponding N-loweralkyl compounds.

GB—A—872447 discloses carbamylazetidines of the formula

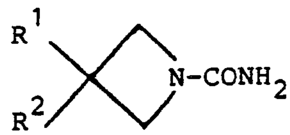

wherein each of $R^1$ and $R^2$ represents hydrogen, ($C_1$—$C_8$) alkyl, cycloalkyl, phenyl or phenylalkyl.

In Houben-Weyl's "Methoden der organischen Chemie" (4th Edn. Vol. VIII, George Thieme Verlag, Stuttgart, 1952) mention is made of the preparation of substituted ureas from urea and acid chlorides or acid anhydrides.

According to a first aspect of the present invention, there are provided 3-phenoxy-1-azetidine carboxamides of the formula:

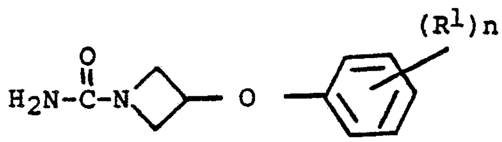

Formula I

wherein

$R^1$ represents hydrogen, fluoro, $C_{1-8}$-alkyl, $C_{1-8}$-alkoxy, trifluoromethyl, acetyl or aminocarbonyl: and n is an integer from 1 to 3 inclusive and when n is 2 or 3 $R^1$ may be the same or different.

In the further definition of symbols in Formula I and where they appear elsewhere throughout this specification and in the claims, the terms have the following significance.

The term "($C_1$—$C_8$)alkyl" includes straight and branched chain hydrocarbon radicals of up to eight carbon atoms inclusive and is exemplified by such groups as methyl, ethyl, propyl, isopropyl, butyl, isobutyl, tertiary butyl, amyl, isoamyl, hexyl, heptyl and octyl.

The term "($C_1$—$C_8$)alkoxy" has the formula O—($C_1$—$C_8$)alkyl.

The compounds of Formula I are useful because of their pharmacological action on the central nervous system.

According to a second aspect of the present invention, there are provided 3-phenoxy-1-azetidine-carboxamides of the formula:

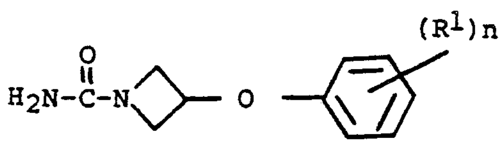

Formula I

wherein

$R^1$ represents hydrogen, fluoro, ($C_1$—$C_8$)alkyl, ($C_1$—$C_8$)alkoxy, trifluoromethyl, acetyl or aminocarbonyl; n is an integer from 1 to 3 inclusive and when n is 2 or 3 $R^1$ may be the same or different

for use in human and/or veterinary medicine, particularly for use in the treatment or prophylaxis of disorders of the central nervous system such as epilepsy.

According to a third aspect of the present invention, there is provided a pharmaceutical composition comprising a) an effective amount of a compound in accordance with the first aspect of the invention and b) a pharmaceutically acceptable carrier therefor.

According to a fourth aspect of the present invention, there is provided a process for the preparation of a compound in accordance with the first aspect of the invention, which process comprises reacting a compound of the formula:

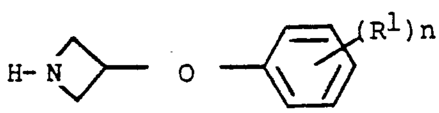

Formula I

wherein $R^1$ and n are as defined in relation to the first aspect, with nitrourea.

Preferred compounds of the first aspect of the invention are those in which n is 1 or 2, especially 1. $R^1$ may represent 2-, 3- or 4- trifluoromethyl, especially 3-trifluoromethyl. $R^1$ may also represent 4-alkoxy, especially 4-methoxy, or di-$C_{1-8}$-alkoxy, especially dimethoxy, such as 3,5-di-$C_{1-8}$-alkoxy. Compounds in which $R^1$ represents 3-fluoro or acetyl, especially 4-acetyl, are also preferred, as are compounds in which $R^1$ represents methyl such as 4-methyl and other 4-alkyl substituents.

Preferred features of the second, third and fourth aspects of the invention are as for preferred features of the first aspect *mutatis mutandis.*

The procedure for testing the compounds for their anticonvulsant activity and comparison with prior art compounds is based on evaluation techniques published by Swinyard, E. A. in EPILEPSIA *10*: 107—19 (1969) and in J. PHARMAC. EXPTL. THERAP. *106*: 319—30 (1952) as explained in greater detail below.

A reaction sequence for the preparation of compounds of Formula I is shown in Chart I. A preparation of certain of the compounds of Formula II is also disclosed in US—A—4379151 filed on April 5, 1983. Compounds of Formula III wherein $R^2$ is α-methylbenzyl or diphenylmethyl can be prepared by reacting compounds of Formula IV and V at temperatures up to about 80—100°C for periods of 2—5 hr in dimethylformamide. Compounds of Formula II can be prepared by hydrogenolysis of compounds of Formula III, usually in the presence of a lower-alkanol solvent, ethanol being preferred. The rate of hydrogenolysis is dependent somewhat on time and temperature, a higher temperature generally decreasing the time required for complete hydrogenolysis. Typical times vary from about 3 hr to about 24 hr at temperatures of 50—90°C.

CHART 1

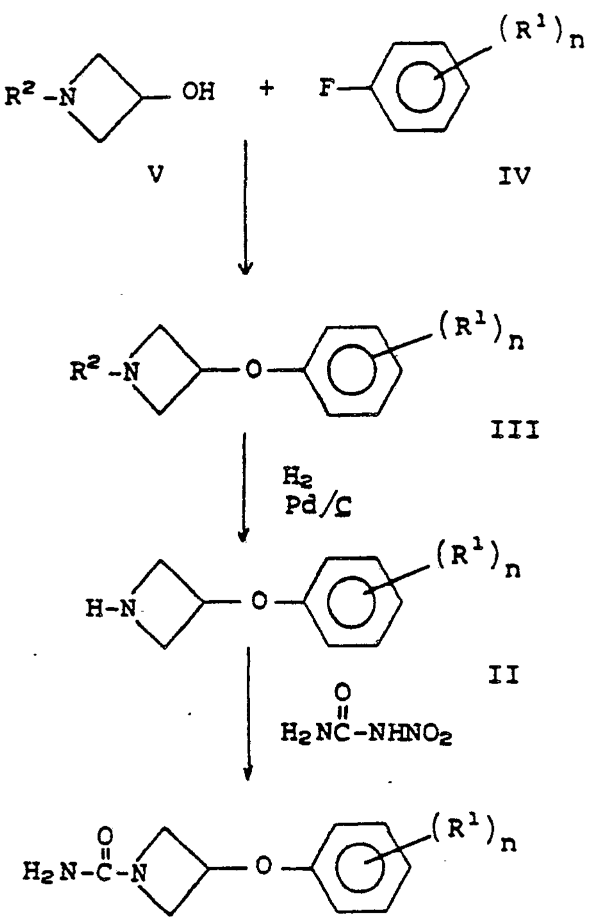


Formula I

$R^2$ = α-methylbenzyl or diphenylmethyl.

$R^1$ = hydrogen, $C_1$—$C_8$-alkyl, $C_1$—$C_8$-alkoxy, fluorine, trifluoromethyl, acetyl or aminocarbonyl.

n is 1—3 wherein $R^1$ may be the same or different.

In the final step, compounds of Formula II can be reacted with nitrourea in solution to give the products (I) conveniently, for example, in a mixture of ethanol and methylene chloride or acetone at room temperature, usually until analysis indicates substantial reaction has occurred. Products are isolated by evaporation of reaction solvent, partitioning with water and an organic solvent for the product and evaporating the organic solvent layer and recrystallizing.

When compounds of Formulae II or III are isolated as an acid addition salt in synthesis procedure and it is desirable to obtain the free base, the salts are partitioned in a dilute aqueous basic solution and a suitable organic solvent for the free base and then the free base is isolated by drying and evaporating the organic solvent.

Preparations 1—18 illustrate the preparation of compounds of Formula II and their precursors and the examples illustrate the final conversion to compounds of Formula I.

Preparation 1

3-(3-Chlorophenoxy-1-(α-methylbenzyl)azetidine Oxalate.

1-(α-Methylbenzyl)-3-hydroxyazetidine maleate (393 g, 1.3 moles) was partitioned in dilute potassium hydroxide-benzene. The separated dried benzene solution was concentrated, the residual oil dissolved in 250 ml. of dimethylformamide and added dropwise to a stirred suspension of 53 g (1.1 moles) of 50% sodium hydride in 750 ml of dimethylformamide at 90°C. The mixture was heated at 90°C for 1 hr and 130.5 g (1 mole) of 3-chlorofluorobenzene added dropwise at 90°C. The mixture was refluxed for 3 hrs, cooled and partitioned between isopropyl ether and dilute sodium hydroxide. The isopropyl ether solution was dried, concentrated, and the residue added to 1200 ml of isopropyl alcohol containing 90 g (1 mole) of oxalic acid. The oxalate salt was recrystallized from ethanol. Yield 263 g (69%); m.p. 141—144°C.

Analysis: Calculated for $C_{19}H_{20}ClNO_5$: C, 60.40; H, 5.34; N, 3.71
Found: C, 60.19; H, 5.55; N, 3.60

Preparation 2

1-(α-Methylbenzyl)-3-(4-trifluoromethylphenoxy)azetidine.

The maleate salt of 1-(α-methylbenzyl)-3-hydroxyazetidine (78.6 g, 0.20 mole) was partitioned between benzene and dilute sodium hydroxide, the benzene layer dried, filtered, and concentrated at reduced pressure. The residue was dissolved in 100 ml of dry dimethylformamide and added at a rapid dropwise rate, to a stirring suspension of 10.1 g (0.22 mole) of sodium hydride (50% in mineral oil) in 150 ml of dry dimethylformamide at 90°C. The solution was heated at 90°C for one hour and then treated dropwise with 32.0 g (0.20 mole) of 4-trifluoromethylfluorobenzene. The solution was refluxed for three hours. The cooled solution was partitioned between water and isopropyl ether, and the ether layer extracted with dilute hydrochloric acid. The aqueous acid layer was made basic with concentrated sodium hydroxide and ice, and extracted with isopropyl ether. The ether layer was concentrated and the residue distilled at 150—160°C/0.2 mm to give 25.6 g of product.

Analysis: Calculated for $C_{18}H_{18}F_3NO$: C, 67.28; H, 5.65; N, 4.36
Found: C, 67.27; H, 5.84; N, 4.34

Preparations 3 to 7 were prepared according to the procedures set forth in detail in Preparations 1 and 2 by reacting 1-(α-methylbenzyl)-3-azetidinol with the appropriately substituted fluorobenzene. The physical constants are shown in Table I.

TABLE I

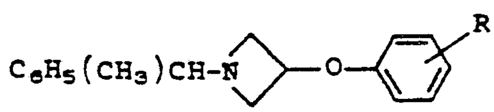

| Preparation | R | M.p. (b.p.) °C | Salt |
|---|---|---|---|
| 3 | 2—$CONH_2$ | 148—52 | — |
| 4 | 4—CN | 65—8 | — |
| 5 | 3—$CF_3$ | 150—3 | $(COOH)_2$ |
| 6 | 2—$CF_3$ | 162—3 | $(COOH)_2$ |
| 7 | 3—CN | [1](185—90) | — |

[1]At 0.2 mm.

The analytical data of Preparations 3 to 7 are shown in Table II.

TABLE II
Analytical Data on Preparations 3 to 7

| Preparation | Empirical Formula | Calculated C | Calculated H | Calculated N | Found C | Found H | Found N |
|---|---|---|---|---|---|---|---|
| 3 | $C_{18}H_{20}N_2O_2$ | 72.95 | 6.80 | 9.45 | 72.56 | 6.78 | 9.32 |
| 4 | $C_{18}H_{18}N_2O$ | 77.67 | 6.52 | 10.06 | 77.61 | 6.53 | 10.01 |
| 5 | $C_{20}H_{20}F_3NO_5$ | 58.39 | 4.90 | 3.41 | 57.99 | 4.97 | 3.39 |
| 6 | $C_{20}H_{20}F_3NO_5$ | 58.39 | 4.90 | 3.41 | 58.15 | 4.89 | 3.37 |
| 7 | $C_{18}H_{18}N_2O$ | 77.67 | 6.52 | 10.06 | 77.32 | 6.54 | 9.87 |

Preparation 8

3-[1-(α-Methylbenzyl)-3-azetidinyloxy]benzamide Oxalate.

3-[1-(α-Methylbenzyl)-3-azetidinyloxy]benzonitrile (50.g g, 0.18 mole) in 500 ml of t-butyl alcohol was treated with 50.0 g of finely ground potassium hydroxide. The mixture was stirred at reflux for 30 min. Ice and water were added to the reaction mixture and the organic layer was separated and dried over sodium sulfate. The dried filtered solution was concentrated at reduced pressure. The residue was dissolved in methanol and treated with an equivalent of oxalic acid, and the oxalate salt was recrystallized from ethanol to give 11.4 g (16%) of product, (m.p. 145°C).

Analysis: Calculated for $C_{20}H_{22}N_2O_6$: C, 62.17; H, 5.74; N, 7.25
Found: C, 62.17; H, 5.80; N, 7.20

Preparation 9

4-[1-(α-Methylbenzyl)-3-azetidinyloxy]benzamide.

To 45.0 g (0.16 mole); of 4-[1-(α-methylbenzyl)-3-azetidinyloxy]benzonitrile in 500 ml of t-butyl alcohol was added 45.0 g of finely ground potassium hydroxide. The mixture was stirred and refluxed for 30 minutes. Ice and water were added and a thick white solid separated. The solid was recrystallized from toluene to give 30.0 g (63%) of product melting at 174—178°C.

Analysis: Calculated for $C_{18}H_{20}N_2O_2$: C, 72.05; H, 6.80; N, 9.45
Found: C, 73.06; H, 6.79; N, 9.44

Preparation 10

1-Diphenylmethyl-3-phenoxyazetidine.

To a stirred suspension of 8.6 g (0.22 mole) of sodium amide in 100 ml of dry toluene was added 18.2 g (0.2 mole) of phenol in 50 ml of dry toluene. After stirring for 2 hrs at 60°C the pot temperature was raised to 80°C and a solution of 1-diphenylmethyl-3-methylsulphonyloxyazetidine (63.4 g, 0.2 mole) in 200 ml of dry toluene was added dropwise. After an additional 2 hrs at 80°C the cooled mixture was treated with water, the toluene layer was extracted with dilute sodium hydroxide solution, dried and concentrated at reduced pressure. The residue was crystallized twice from a water-isopropanol mixture. The free base melted at 83—85°C.

Analysis: Calculated for $C_{22}H_{21}NO$: C, 83.78; H, 6.71; N, 4.44
Found: C, 83.69; H, 6.81; N, 4.41

Preparation 11

3-(Phenoxy)azetidine Methanesulphonate

A 200 ml solution of 7.8 g (0.025 mole) of 1-diphenylmethyl-3-phenoxyazetidine in ethanol was treated with 20% Pd $(OH)_2$ on carbon and hydrogenated for 23 hrs at about 45 psi and 80°C. The mixture was filtered and the filtrate concentrated. The residue was diluted to 30 ml with ethanol and 2.5 g of methanesulphonic acid added. The isolated methanesulphonate salt was recrystallized from ethanol. The salt weighed 2.3 g (37.5%) and melted at 128—130°C.

Analysis: Calculated for $C_{10}H_{15}NO_4S$: C, 48.97; H, 6.16; N, 5.71
Found: C, 48.40; H, 6.19; N, 5.63

The compound was also prepared by hydrogenolysis of 1-(α-methylbenzyl)-3-(3-chlorophenoxy)azetidine in isopropyl alcohol using the same type catalyst and conditions.

Preparation 12

3-[4-(Trifluoromethyl)phenoxy]azetidine Oxalate.

To 24.0 g (0.075 mole) of 3-[4-(trifluoromethyl)phenoxy]-1(α-methylbenzyl)azetidine in 150 ml of ethanol was added 0.5 g of 20% $Pd(OH)_2$ on carbon, and the mixture was hydrogenated for five hours at 80°C and 45 psi. The mixture was cooled, filtered, and the filtrate concentrated at reduced pressure. The residue was dissolved in ethanol and treated with oxalic acid, and the oxalate salt was recrystallized three times in ethanol. The yield was 3.0 g (13%) and the salt melted at 176—178°C.

Analysis: Calculated for $C_{12}H_{12}F_3NO_3$: C, 46.91; H, 3.94; N, 4.56
Found: C, 47.07; H, 3.96; N, 4.59

The compounds in Preparations 13 to 17 are prepared according to the procedure set forth in detail in Preparations 11 and 12 by hydrogenolysis of the α-methylbenzyl radical attached to the azetidine nitrogen. The physical constants are shown in Table 1.

TABLE 1

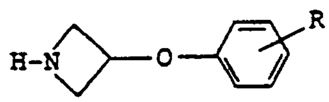

| Preparation | R | M.p. °C | Salt |
|---|---|---|---|
| 13 | 2—$CONH_2$ | 173—75 | $CH_3SO_3H$ |
| 14 | 3—$CF_3$ | 123—25 | [1]$C_6H_{11}NHSO_3H$ |
| 15 | 2—$CF_3$ | 154—56 | HCl |
| 16 | 3—$CONH_2$ | 160—63 | — |
| 17 | 4—$CONH_2$ | 187—88 | $(COOH)_2$ |

[1]N-cyclohexylsulphamate.

The analytical data of Preparations 13 to 17 are shown in Table 2.

TABLE 2
Analytical Data on Preparations 13 to 17

| Preparation | Empirical Formula | Calculated C | Calculated H | Calculated N | Found C | Found H | Found N |
|---|---|---|---|---|---|---|---|
| 13 | $C_{11}H_{16}N_2O_5S$ | 45.42 | 5.59 | 9.72 | 45.48 | 5.65 | 9.45 |
| 14 | $C_{16}H_{23}F_3N_2O_4S$ | 48.48 | 5.85 | 7.07 | 48.08 | 5.94 | 6.97 |
| 15 | $C_{10}H_{11}ClF_3NO$ | 47.35 | 4.37 | 5.52 | 47.12 | 4.32 | 5.45 |
| 16 | $C_{10}H_{12}N_2O_2$ | 62.49 | 6.29 | 14.57 | 62.06 | 6.13 | 13.98 |
| 17 | $C_{12}H_{14}N_2O_6$ | 51.07 | 5.00 | 9.93 | 51.39 | 5.22 | 9.56 |

Preparation 18

When in the procedure of Preparation 2 the following are substituted for 3-[4-(trifluoromethyl)phenoxy]-1-(α-methylbenzyl)azetidine:

3-[4-(methyl)phenoxy]-1-(α-methylbenzyl)azetidine,
3-[4-(methoxy)phenoxy]-1-(α-methylbenzyl)azetidine,
3-[3,5-(dimethoxy)phenoxy]-1-(α-methylbenzyl)azetidine,
3-[3-(fluoro)phenoxy]-1-(α-methylbenzyl)azetidine, and
3-[4-(acetyl)phenoxy]-1-(α-methylbenzyl)azetidine, there are obtained:
3-[4-(methyl)phenoxy]azetidine oxalate,

3-[4-(methoxy)phenoxy]azetidine oxalate,
3-[3,5-(dimethoxy)phenoxy]azetidine oxalate,
3-[3-(fluoro)phenoxy]azetidine oxalate, and
3-[4-(acetyl)phenoxy]azetidine oxalate.

Example 1

3-[3-Trifluoromethyl)phenoxy]-1-azetidinecarboxamide.

To a solution of 2.2 g (0.01 mole) of 3-[3-(trifluoromethyl)phenoxy]azetidine in 45 ml of methylene chloride and 45 ml of absolute ethyl alcohol was added 7 g (0.066 mole) of nitrourea and the mixture was stirred at room temperature for 48 hr. The mixture was filtered. The filtrate was evaporated to dryness and the residue was partitioned between 75 ml methylene chloride and 75 ml water. The water layer was extracted three times with 50 ml of methylene chloride. The methylene chloride extracts were combined and evaporated to dryness. The residue was treated (washed) with a mixture of 1 ml methylene chloride and 20 ml of toluene and filtered. The precipitate was recrystallized from ethanol-water to give pale yellow crystals. The crystals were mixed with 2 ml of methylene chloride and 20 ml toluene and the mixture was heated on a steam bath for 2 hrs. The mixture was stored in a refrigerator for approximately 72 hrs and filtered to give 1.2 g of the product as white crystalline needles, m.p. 151—152°C.

| | | | |
|---|---|---|---|
| Analysis: Calculated for $C_{11}H_{11}N_2O_2F_3$: | C, 50.77; | H, 4.26; | N, 10.77 |
| Found: | C, 50.72; | H, 4.25; | N, 10.74 |

Example 2

3-[3-(Trifluoromethyl)phenoxy]-1-azetidinecarboxamide.

A mixture of 30.6 g (0.141 mole) of 3-[3-(trifluoromethyl)phenoxy]azetidine and 42 g (0.321 mole) of nitrourea (80%) in 500 ml of acetone was stirred for 5 days (5 days not required, but convenient) at room temperature. The mixture was filtered and the filtrate concentrated *in vacuo*. The residue was partitioned between 150 ml of water and 100 ml of ethyl acetate and the layers separated. The aqueous layer was washed with 100 ml of ethyl acetate. The ethyl acetate layers were washed with 75 ml of 5% aqueous sodium hydroxide solution followed by 75 ml of water, dried over sodium sulphate and concentrated *in vacuo*. The residual oil was crystallized from ethyl alcohol-ethyl acetate to give 22 g (60%) substantially the title compound. Recrystallization twice from ethyl alcohol gave 9.9 g of white crystalline solid, m.p. 151—152.5°C.

| | | | |
|---|---|---|---|
| Analysis: Calculated for $C_{11}H_{11}N_2O_2F_3$: | C, 50.77; | H, 4.26; | N, 10.76 |
| Found: | C, 50.90; | H, 4.29; | N, 10.71 |

Example 3

When in the procedure of Example 2 the following are substituted for 3-[3-(trifluoromethyl)phenoxy]azetidine:

3-(phenoxy)azetidine,
3-[4-(trifluoromethyl)phenoxy]azetidine,
3-[2-(trifluoromethyl)phenoxy]azetidine,
3-[4-(methyl)phenoxy]azetidine,
3-[4-(methoxy)phenoxy]azetidine,
3-[3,5-(dimethoxy)phenoxy]azetidine,
3-[3-(fluoro)phenoxy]azetidine,
2-(3-azetidinyloxy)benzamide,
3-(3-azetidinyloxy)benzamide,
4-(3-azetidinyloxy)benzamide, and
3-[4-(acetyl)phenoxy]azetidine,

there are obtained:

3-(phenoxy)-1-azetidinecarboxamide,
3-[4-(trifluoromethyl)phenoxy]-1-azetidinecarboxamide,
3-[2-(trifluoromethyl)phenoxy]-1-azetidinecarboxamide,
3-[4-(methyl)phenoxy]-1-azetidinecarboxamide,
3-[4-(methoxy)phenoxy]-1-azetidinecarboxamide,
3-[3,5-(dimethoxy)phenoxy]-1-azetidinecarboxamide,
3-[3-(fluoro)phenoxy]-1-azetidinecarboxamide,
3-[2-(carboxamido)phenoxy]-1-azetidinecarboxamide,
3-[3-(carboxamido)phenoxy]-1-azetidinecarboxamide,
3-[4-(carboxamido)phenoxy]-1-azetidinecarboxamide, and
3-[4-(aceto)phenoxy]-1-azetidinecarboxamide.

*Pharmacology*

A comparison was made of anti-convulsant activity of the compound of Example 1 and the prior art methyl analogue (U.S. 4,226,861, Example 4) using Metrazole as the convulsant by the method of Swinyard (See above citation).

Ninety-six adult female mice weighing 24—32 g were randomly assigned to dosage groups according to the method of Steel, R.G.D. and Torrie, J.H. (1960) in "Principles and Procedures of Statistics", McGraw-Hill Book Company, Inc., pp 99—100, pp 428—31. Each mouse was identified with a colour code on its tail. The test compounds were administered as suspensions in 10 ml/kg mouse body weight of 0.5% aqueous methyl cellulose within 15 minutes of preparation of the suspension. Metrazole (pentylenetetrazol) was prepared as a solution in physiological saline. The mice were not fasted prior to the test. Eight mice were tested at each dosage level.

Each mouse received one dose of the test drug in the 0.5% aqueous methylcellulose or the control article (0.5% aqueous methylcellulose alone) intraperitoneally. Observation for loss of righting was made ½ hour after administration of test drug. Metrazole (80 mg/kg S.C.) was then given in a loose fold of skin on the back of the neck; i.e., ½ hr after the test compound or control article was given. Injections were given with a 1-ml glass tuberculin syringe with appropriate size hypodermic needle (27 gauge for solutions; 23 gauge for suspensions). All injections were give in a volume of 10 ml/kg mouse body weight. Each mouse was observed for 30 minutes following Metrazole injection. Failure of the animals to exhibit a threshold seizure (a single episode of clonic spasms at least 5 seconds in duration) was defined as protection. Anticonvulsant data were tabulated as the percent protection, i.e.,

$$\frac{\text{No. Mice Protected}}{\text{No. Mice Tested}} \times 100$$

The $ED_{50}$ and (95% confidence limits) and potency ratio were ascertained by the computer-based probit analysis ascribed to Finney, D. J. (1964) *Statistical Method in Biological Assay.,* 2nd Ed., New York, Hafner Publishing Co. Results are in Table A. Statistical analysis of the data indicated no significant difference in anti-convulsant potency between the two compounds.

TABLE A
Muscle Relaxant Activity In Mice

| Test Compound (a) (In Methylcellulose Suspension) Name | Dose, i.p. mq/kq | Number with Loss of Righting / Number Tested (b) |
|---|---|---|
| Control-Methylcellulose alone | none | 0/24 |
| 3-[3-(Trifluoromethyl)phenoxy]-1-azetidinecarboxamide (Example 1) | 45 | 0/8 |
| | 67 | 0/8 |
| | 100 | 0/8 |
| | 150 | 0/8 |
| | 225 | 0/8—(some limb weakness) |
| Prior art compound— | | |
| N-Methyl-3-(3-trifluoromethyl-phenoxy-1-azetidinecarboxamide (Example 4 of US—A—4,226,861) | 45 | 0/8 |
| | 67 | 0/8 |
| | 100 | 3/8 |
| | 150 | 5/8 |

Footnotes:

a) All mice were injected with 10 ml/kg of 0.5% aqueous methylcellulose intraperitoneally in which test compound was suspended if present

b) After 30 minutes.

TABLE A
Anticonvulsant Activity In Mice (b)

| Test Compound (a) (In Methylcellulose Suspension) Name | Dose, i.p. mg/kg | Number Protected / Number Tested | $ED_{50}$ and (Confidence Limits) mg/kg | Potency and 95% Confidence Limits |
|---|---|---|---|---|
| Control-Methylcellulose alone | none | 0/24 | — | |
| 3-[3-(Trifluoromethyl)phenoxy]-1-azetidinecarboxamide (Example 1) | 45 | 1/8 | 99.2 (66.7—147.5) | 0.92 (0.62—1.38) |
| | 67 | 2/8 | | |
| | 100 | 4/8 | | |
| | 150 | 5/8 | | |
| | 225 | 8/8 | | |
| Prior art Compound— N-Methyl-3-(3-trifluoromethyl-phenoxy)-1-azetidinecarboxamide (Example 4 of US—A—4226861) | 45 | 1/8 | 90.9 (61.7—137.9) | 1.00 |
| | 67 | 2/8 | | |
| | 100 | 3/8 | | |
| | 150 | 8/8 | | |

Footnotes:

a) All mice were first injected with 10 ml/kg of 0.5% aqueous methylcellulose intraperitoneally in which the test compound was suspended if present.

b) 80 mg/kg Metrazole in 10 ml saline of mouse body weight was administered to all mice subcutaneously in a loose fold of skin on the back of the neck one-half hr after administration of test compound.

*Formulation and Administration*

The pharmacologically active 3-phenoxy-1-azetidine carboxamides of this invention are effective in the treatment of both petit mal epilepsy and grand mal epilepsy. Effective quantities of these compounds may be administered to a living animal body orally as in capsules, tablets or elixirs. It is only necessary that the active ingredient constitute an effective amount, i.e., such that a suitable effective dosage will be obtained consistent with the dosage form employed. The exact individual dosage as well as daily dosages will, of course, be determined according to standard medical principles under the direction of a physician or veterinarian.

Based upon a comparison with known anticonvulsant compounds, preferred daily dosages appear to range from about 0.5 to 1.5 milligrams per kilogram of body weight in the treatment of petit mal epilepsy and about 25 to 35 milligrams per kilogram of body weight in the treatment of grand mal epilepsy. Very small quantities of the active materials of the present invention, even as low as 0.1 milligram, are effective when minor therapy is involved. Unit dosages are usually 5 milligrams or above and preferably 25, 50 or 100 milligrams per unit dose. The active ingredients of the invention may be combined with other pharmacologically active agents as previously indicated, or with buffers, antacids or the like, for administration and the proportion of the active agent in the composition may be varied widely.

*Capsules*

Capsules of 5 mg, 25 mg, and 50 mg of active ingredient per capsule are prepared; with higher amounts of ingredient reduction may be made in the amount of lactose.

| Typical blend for encapsulation | Per Capsule, mg. |
|---|---|
| Active ingredient | 5.0 |
| Lactose | 296.7 |
| Starch | 129.0 |
| Magnesium stearate | 4.3 |
| Total | 435.0 mg. |

Uniformly blend the selected active ingredient with lactose, starch and magnesium stearate and encapsulate the blend.

Additional capsule formulations preferably contain a higher dose of active ingredient and are as follows:

| Ingredients | 100 mg per Capsule | 250 mg per Capsule | 500 mg per Capsule |
|---|---|---|---|
| Active ingredient | 100.0 | 250.0 | 500.0 |
| Lactose | 231.5 | 126.5 | 31.1 |
| Starch | 99.2 | 54.2 | 13.4 |
| Magnesium stearate | 4.3 | 4.3 | 5.5 |
| Total, mg | 435.0 | 435.0 | 550.0 |

*Tablets*

A typical formulation for a tablet containing 5.0 mg of active ingredient per tablet follows. The formulation may be used for other strengths of active ingredient by adjustment of weight of dicalcium phosphate.

| Ingredients | Per Tablet, mg |
|---|---|
| (1) Active ingredient | 5.0 |
| (2) Corn Starch | 13.6 |
| (3) Corn Starch (paste) | 3.4 |
| (4) Lactose | 79.2 |
| (5) Dicalcium phosphate | 68.0 |
| (6) Calcium Stearate | 0.9 |
| Total | 170.1 mg. |

Uniformly blend 1, 2, 4 and 5. Prepare 3 as a 10 percent paste in water. Granulate the blend with the starch paste and pass the wet mass through a number eight mesh screen. The wet granulation is dried and passed through a number twelve mesh screen. The dried granules are blended with calcium stearate and compressed.

Additional tablet formulations preferably contain a higher dosage of the active ingredient and are as follows:

50 mg Tablet

| Ingredients | Per Tablet, mg |
|---|---|
| Active ingredient | 50.0 |
| Lactose | 90.0 |
| Milo starch | 20.0 |
| Corn starch | 38.0 |
| Calcium stearate | 2.0 |
| Total | 200.0 |

Uniformly blend the active ingredient, lactose, milo starch and corn starch. The blend is granulated, using water as a granulating medium. The wet granules are passed through an eight mesh screen and dried at 60 to 71 degrees celcius (140 to 160 degrees Fahrenheit) overnight. The dried granules are passed through a number ten mesh screen and blended with the proper amount of calcium stearate and this blend is then converted into tablets on a suitable tablet press.

**Claims**

1. A compound characterised by the formula:

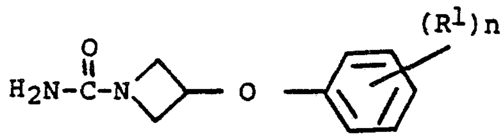

wherein

$R^1$ represents hydrogen, fluoro, $(C_1—C_8)$alkyl, $(C_1—C_8)$alkoxy, trifluoromethyl, acetyl or aminocarbonyl; and

n is an integer from 1 to 3 inclusive and when n is 2 or 3 $R^1$ may be the same or different.

2. A compound as claimed in Claim 1, wherein $R^1$ represents a substituent in the 3-position.

3. A compound as claimed in Claim 1 or 2, wherein $R^1$ represents trifluoromethyl.

4. 3-[3-(Trifluoromethyl)phenoxy]-1-azetidinecarboxamide.

5. A compound as claimed in Claim 1, 2 or 3 for use in medicine.

6. 3-[3-(Trifluoromethyl)phenoxy]-1-azetidinecarboxamide for use in medicine.

7. A pharmaceutical composition comprising a) an effective amount of a compound characterised by the formula:

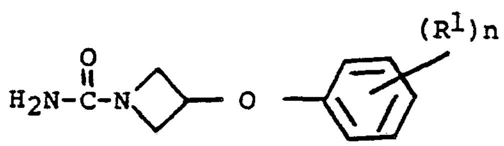

wherein

$R^1$ represents hydrogen, fluoro, $(C_1—C_8)$alkyl, $(C_1—C_8)$alkoxy, trifluoromethyl, acetyl or aminocarbonyl; and

n is an integer from 1 to 3 inclusive and when n is 2 or 3 $R^1$ may be the same or different, and

b) a pharmaceutically acceptable carrier therefor.

8. A pharmaceutical composition comprising 3-[3-(trifluoromethyl)phenoxy]-1-azetidinecarboxamide, and a pharmaceutically acceptable carrier therefor.

9. A process for the preparation of a compound characterised by the formula:

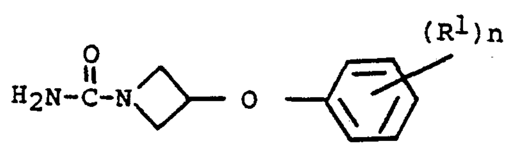

wherein

$R^1$ represents hydrogen, fluoro, $(C_1—C_8)$alkyl, $(C_1—C_8)$alkoxy, trifluoromethyl, acetyl or aminocarbonyl; and

n is an integer from 1 to 3 inclusive and when n is 2 or 3 $R^1$ may be the same or different, which process comprises reacting a compound of the formula:

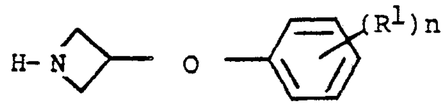

wherein $R^1$ and n are as defined above, with nitrourea.

10. A process for the preparation of 3-[3-(trifluoromethyl)phenoxy]-1-azetidinecarboxamide, comprising reacting 3-[3-(trifluoromethyl)phenoxy]azetidine with nitrourea.

**Patentansprüche**

1. Verbindung, gekennzeichnet durch die Formel

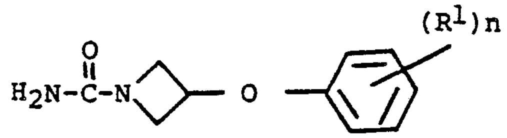

worin $R^1$ Wasserstoff, Fluor, $(C_1—C_8)$-Alkyl, $(C_1—C_8)$-Alkoxy, Trifluormethyl, Acetyl oder Aminocarbonyl darstellt, und n eine ganze Zahl von 1 bis einschließlich 3 ist, wobei dann, wenn n gleich 2 oder 3 ist, $R^1$ gleich oder verschieden sein kann.

2. Verbindung nach Anspruch 1, worin $R^1$ einen Substituenten in der 3-Stellung darstellt.

3. Verbindung nach Anspruch 1 oder 2, worin $R^1$ Trifluormethyl darstellt.

4. 3-[3-(Trifluormethyl)-phenoxy]-1-azetidin-carboxamid.

5. Verbindung nach Anspruch 1, 2 oder 3, zur Verwendung in der Medizin.

6. 3-[3-(Trifluormethyl)-phenoxy]-1-azetidin-carboxamid zur Verwendung in der Medizin.

7. Pharmazeutische Zusammensetzung, umfassend

a) eine wirksame Menge einer Verbindung der Formel

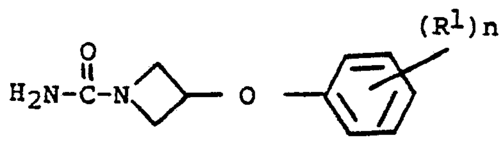

worin $R^1$ Wasserstoff, Fluor, $(C_1—C_8)$-Alkyl, $(C_1—C_8)$-Alkoxy, Trifluormethyl, Acetyl oder Aminocarbonyl darstellt, und n eine ganze Zahl von 1 bis einschließlich 3 ist, wobei dann, wenn n gleich 2 oder 3 ist, $R^1$ gleich oder verschieden sein kann; und

b) einen pharmazeutisch annehmbaren Träger hiefür.

8. Pharmazeutisch Zusammensetzung, umfassend 3-[3-(Trifluormethyl)-phenoxy]-1-azetidincarboxamid und einen pharmazeutisch annehmbaren Träger hiefür.

9. Verfahren zur Herstellung einer Verbindung der Formel

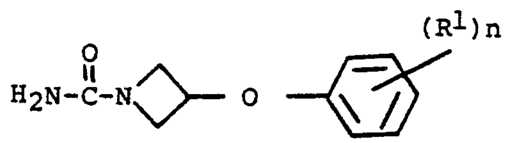

worin $R^1$ Wasserstoff, Fluor, $(C_1—C_8)$-Alkyl, $(C_1—C_8)$-Alkoxy, Trifluormethyl, Acetyl oder Aminocarbonyl darstellt, und n eine ganze Zahl von 1 bis einschließlich 3 ist, wobei dann, wenn n gleich 2 oder 3 ist, $R^1$ gleich oder verschieden sein kann, welches Verfahren die Umsetzung einer Verbindung der Formel

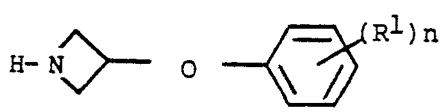

worin $R^1$ und n wie vorher definiert sind, mit Nitroharnstoff umfaßt.

10. Verfahren zur Herstellung von 3-[3-(Trifluormethyl)phenoxy]-1-azetidincarboxamid, umfassend die Umsetzung von 3-[3-(Trifluormethyl)-phenoxy]-azetidin mit Nitroharnstoff.

**Revendications**

1. Composé caractérisé par la formula:

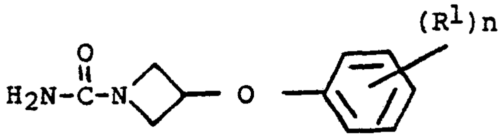

où $R^1$ représente un atome d'hydrogène ou radical fluoro, ($C_1$—$C_8$)alcoyle, ($C_1$—$C_8$)alcoxy, trifluorométhyle, acétyle ou aminocarbonyle, et

n est un nombre entier de 1 à 3 inclusivement et lorsque n est 2 ou 3, les $R^1$ peuvent être identiques ou différents.

2. Composé suivant la revendication 1, dans lequel $R^1$ représente un substituant à la position 3.

3. Composé suivant la revendication 1 ou 2, dans lequel $R^1$ représente un radical trifluorométhyle.

4. Le 3-[3-(trifluorométhyl)phénoxy]-1-azétidinecarboxamide.

5. Composé suivant la revendication 1, 2 ou 3 à utiliser en médecine.

6. Le 3-[3-(trifluorométhyl)phénoxy]-1-azétidinecarboxamide à utiliser en médecine.

7. Composition pharmaceutique comprenant

a) une quantité efficace d'un composé caractérisé par la formule:

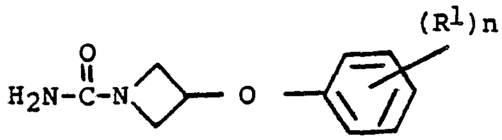

où $R^1$ représente un atome d'hydrogène ou radical fluoro, ($C_1$—$C_8$)alcoyle, ($C_1$—$C_8$)alcoxy, trifluorométhyle, acétyle ou aminocarbonyle, et

n est un nombre entier de 1 à 3 inclusivement et lorsque n est 2 ou 3, les $R^1$ peuvent être identiques ou différents, et

b) un excipient pharmaceutiquement acceptable.

8. Composition pharmaceutique comprenant du 3-[3-(trifluorométhyl)phénoxy]-1-azétidinecarboxamide, et un excipient pharmaceutiquement acceptable.

9. Procédé de préparation d'un composé caractérisé par la formule:

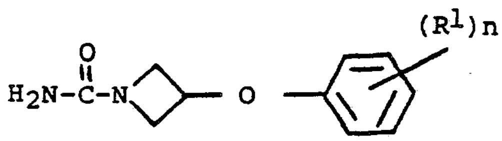

où $R^1$ représente un atome d'hydrogène ou radical fluoro, ($C_1$—$C_8$)alcoyle, ($C_1$—$C_8$)alcoxy, trifluorométhyle, acétyle ou aminocarbonyle, et

n est un nombre entier de 1 à 3 inclusivement et lorsque n est 2 ou 3, les $R^1$ peuvent être identiques ou différents, lequel procédé comprend la réaction d'un composé de formule:

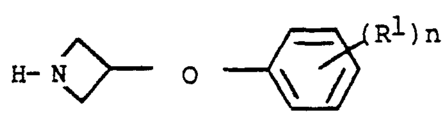

où $R^1$ et n sont tels que définis ci-dessus, avec la nitrourée.

10. Procédé de préparation du 3-[3-(trifluorométhyl)phénoxy]-1-azétidinecarboxamide qui comprend la réaction de la 3-[3-(trifluorométhyl)phénoxy]azétidine avec la nitrourée.